(19) Europäisches Patentamt
European Patent Office
Office européen des brevets

(11) **EP 3 974 001 A1**

(12) **EUROPEAN PATENT APPLICATION**
published in accordance with Art. 153(4) EPC

(43) Date of publication:
**30.03.2022 Bulletin 2022/13**

(21) Application number: **20813403.1**

(22) Date of filing: **28.05.2020**

(51) International Patent Classification (IPC):
***A61L 2/08*** *(2006.01)*

(52) Cooperative Patent Classification (CPC):
**A61L 2/08**

(86) International application number:
**PCT/JP2020/021127**

(87) International publication number:
**WO 2020/241758 (03.12.2020 Gazette 2020/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.05.2019 JP 2019099851**

(71) Applicant: **Hitachi Zosen Corporation
Osaka-shi, Osaka 559-8559 (JP)**

(72) Inventors:
• **TANAKA Ryuta
Osaka-shi, Osaka 559-8559 (JP)**
• **YAMADA Hiroki
Osaka-shi, Osaka 559-8559 (JP)**
• **SAKAI Ichiro
Osaka-shi, Osaka 559-8559 (JP)**

(74) Representative: **Canzler & Bergmeier
Patentanwälte
Partnerschaft mbB
Despag-Strasse 6
85055 Ingolstadt (DE)**

(54) **ELECTRON BEAM STERILIZATION DEVICE**

(57) An electron beam sterilizer (10) includes at least one wire rod (3) configured to hold a sterilization object (O), the wire rod (3) being in a constant relative position with respect to the sterilization object (O); and an electron beam irradiator configured to irradiate the sterilization object (O) held by the wire rod (3) with an electron beam (12) over the wire rod (3). The wire rod (3) includes a curved surface (4) in contact with the sterilization object (O) to hold. Electrons (1) of the electron beam (12) from the electron beam irradiator, and secondary electrons (2) excited by the electrons (1) come around to the curved surface (4) in contact with the sterilization object (O) to sterilize the sterilization object (O).

F I G. 2

**EP 3 974 001 A1**

**Description**

Technical Field

The present invention relates to an electron beam sterilizer.

Background Art

Electron beam sterilizers sterilize an object to be sterilized by irradiating the object with an electron beam. Such electron beam sterilizers have sterilized the object in a short time, and therefore, application thereof is expected in various fields. The electron beam sterilizers have been used for sterilizing objects that require high sterilization accuracy, such as food and drink containers or medical containers. For example, Japanese Patent Laid-Open No. Hei 11-19190 (hereinafter, Patent Literature 1) discloses an electron beam sterilizer for sterilizing a beverage container (PET bottle 2). The electron beam sterilizer holds the PET bottles subjected to electron beam irradiation with two wires (wires 51), and moves the wires. Here, some parts of the PET bottles are not subjected to enough electron beam irradiation. Therefore, moving the two wires causes the parts of the PET bottles to change the positions. Consequently, the PET bottles are fully irradiated with the electron beam.

Summary of Invention

Technical Problem

The electron beam sterilizer in Patent Literature 1 irradiates the entire surface of each PET bottle with the electron beam, as described above. Therefore, high sterilization effect seems to be obtained. However, the electron beam sterilizer needs to move the wires in contact with the PET bottles of the objects to be sterilized relative to the PET bottles. Such relative movement may contaminate the PET bottles, causing insufficient sterilization. Therefore, reducing the risk of contamination is highly desired particularly in the medical field where extremely high accuracy of sterilization is required.

An object of the present invention is to provide an electron beam sterilizer capable of enhancing the effect of sterilization.

Solution to Problem

In order to solve the problem, an electron beam sterilizer according to a first invention includes:

at least one wire rod configured to hold a sterilization object, the wire rod being in a constant relative position with respect to the sterilization object; and

an electron beam irradiator configured to irradiate the sterilization object held by the wire rod with an electron beam over the wire rod,

wherein the wire rod includes a curved surface in contact with the sterilization object to hold, and electrons of the electron beam from the electron beam irradiator, and secondary electrons excited by the electrons come around to the curved surface in contact with the sterilization object to sterilize the sterilization object.

An electron beam sterilizer according to a second invention, in the electron beam sterilizer according to the first invention, further includes a mesh configured to hold the sterilization object by placing the sterilization object on the mesh, or inserting the sterilization object in the mesh,

wherein the mesh includes the wire rod woven together.

An electron beam sterilizer according to a third invention, in the electron beam sterilizer according to the first invention, further includes a brush member configured to hold the sterilization object by placing the sterilization object on the brush member,

wherein the brush member includes the wire rod extending along a direction in which the electron beam is emitted from the electron beam irradiator.

An electron beam sterilizer according to a fourth invention, in the electron beam sterilizer according to the first invention, wherein the wire rod holds the sterilization object by hanging the sterilization object.

An electron beam sterilizer according to a fifth invention, in the electron beam sterilizer according to the first invention, further includes:

a needle member configured to hold the sterilization object by placing the sterilization object on a tip of the needle member; and
a circular member configured to hold the sterilization object by surrounding a side surface of the sterilization object on the tip of the needle member,
wherein the needle member and the circular member include the wire rod.

An electron beam sterilizer according to a sixth invention, in the electron beam sterilizer according to any one of the first to fifth inventions, further includes a rotating mechanism configured to rotate the sterilization object and the wire rod with respect to the electron beam irradiator.

An electron beam sterilizer according to a seventh invention, in the electron beam sterilizer according to the sixth invention, wherein the electron beam irradiator irradiates the side surface of the sterilization object with the electron beam.

An electron beam sterilizer according to an eighth invention, in the electron beam sterilizer according to the seventh invention, further includes an elevating mechanism configured to move the sterilization object and the wire rod up and down with respect to the electron beam irradiator.

Advantageous Effects of Invention

The above electron beam sterilizer can enhance the effect of sterilization. This is because a sufficient number of electrons reach the object to be sterilized without being blocked by the wire rod.

Brief Description of Drawings

[FIG. 1] FIG. 1 is a schematic cross-sectional view illustrating an electron beam sterilizer according to Embodiment of the present invention.
[FIG. 2] FIG. 2 is a schematic enlarged sectional view of the electron beam sterilizer.
[FIG. 3] FIG. 3 is a perspective view of an electron beam sterilizer according to Example 1 of the present invention.
[FIG. 4] FIG. 4 is a perspective view of an electron beam sterilizer according to Example 2 of the present invention.
[FIG. 5] FIG. 5 is a side view of an electron beam sterilizer according to Example 3 of the present invention.
[FIG. 6] FIG. 6 is a perspective view of an electron beam sterilizer according to Example 4 of the present invention.
[FIG. 7] FIG. 7 is a perspective view of an electron beam sterilizer according to Example 5 of the present invention.
[FIG. 8] FIG. 8 is a perspective view of an electron beam sterilizer according to Example 6 of the present invention.
[FIG. 9] FIG. 9 is a perspective view illustrating a modification of the electron beam sterilizer according to Example 4 of the present invention.

Description of Embodiment

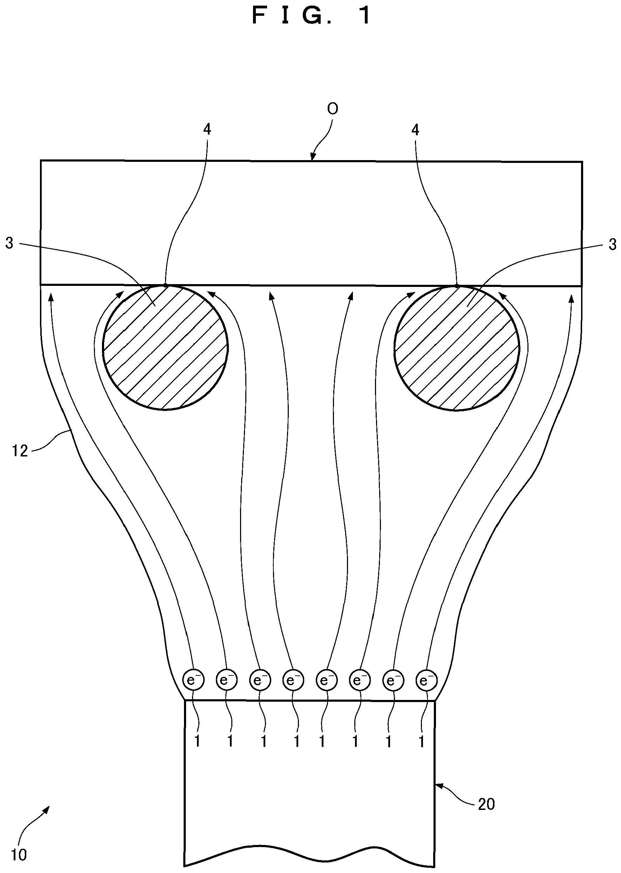

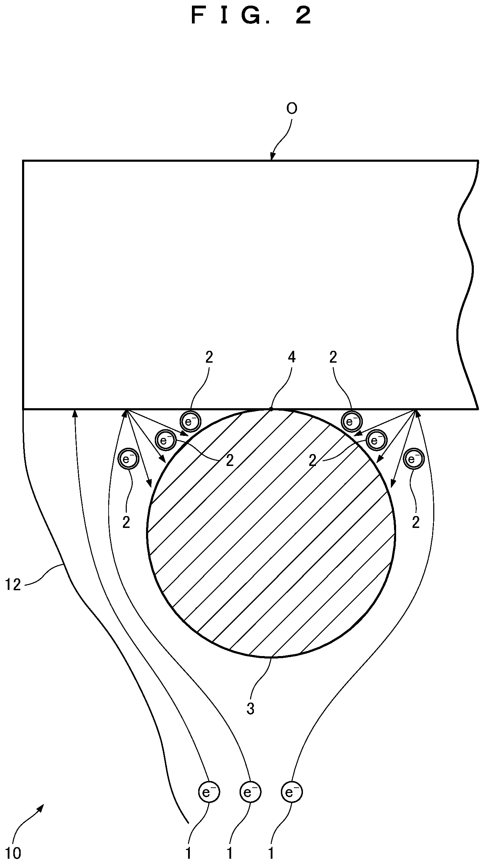

An electron beam sterilizer according to Embodiment of the present invention will be described below in accordance with FIGS. 1 and 2. The electron beam sterilizer sterilizes an object to be sterilized by electron beam irradiation.

As illustrated in FIG. 1, an electron beam sterilizer 10 includes wire rods 3 and an electron beam irradiator 20. The wire rods 3 hold a sterilization object O. The position of each wire rod 3 relative to the sterilization object O is constant. The electron beam irradiator 20 irradiates the sterilization object O held by the wire rods 3 with an electron beam 12 over the wire rods 3.

Each of the wire rods 3 holds the sterilization object O in a constant relative position. In other words, each of the wire rods 3 relative to the sterilization object O is constant with the sterilization object O being held. Further, to hold the sterilization object O, each of the wire rods 3 has a curved surface 4 in contact with the sterilization object O. That is, the wire rods 3 hold the sterilization object O with the curved surfaces 4 by bringing the sterilization object O in contact with such curved surfaces.

Electrons 1 of the electron beam 12 from the electron beam irradiator 20 collide with air molecules to scatter. This causes the electrons 1 to come around to the curved surfaces 4 and reach the parts of sterilization object O in contact with the curved surfaces 4. Consequently, the sterilization object O is sterilized. Further, as illustrated in FIG. 2, which is an enlarged view of FIG. 1, the electrons 1 reach the sterilization object O. Then, secondary electrons 2 of the sterilization object O are excited. The excited secondary electrons 2 are emitted from the sterilization object O. Such secondary electrons 2 also come around to the curved surface 4 and reach the part of sterilization object O in contact with the curved surface 4. Consequently, the sterilization object O is sterilized. The secondary electrons 2 emitted from the sterilization object O are not limited to the electrons excited by the electrons 1 from the electron beam irradiator 20 (hereinafter, may be referred to as primary electrons 1). The secondary electrons 2 include electrons, each excited by the other secondary electrons 2. The secondary electrons 2 emitted from the sterilization object O have lower energy than the primary electrons 1 from the electron beam irradiator 20. The secondary electrons 2 are thus easily absorbed by the bacteria. This enables the secondary electrons 2 easily to sterilize the bacteria. Therefore, the secondary electrons 2 have a much higher sterilization effect than the primary electrons 1.

The wire rod 3 as a holder of the sterilization object O allows the electrons 1 (primary electrons 1) from the electron beam irradiator 20 to easily reach the sterilization object O. This is because the wire rod 3 is less likely to block the primary electrons 1. Such primary electrons 1 excite a large number of the secondary electrons 2 having a high sterilization effect. Then, the secondary electrons 2 are emitted from the sterilization object O. Additionally, the sterilization object O is held by contacting the curved surface 4 of the wire rod 3. This allows the primary electrons 1 and the secondary electrons 2 to easily come around to the part of sterilization object O in contact with the wire rod 3. As a result, a sufficient number of the primary electrons 1 and secondary electrons 2 reach the part of the sterilization object O in contact with the wire rod 3, as well as an area close to the contact part.

As described above, the electron beam sterilizer 10 enables a sufficient number of the primary electrons 1 and secondary electrons 2 to reach the sterilization object O without being blocked by the wire rods 3. Thus, the effect of sterilization can be enhanced.

Hereinafter, an electron beam sterilizer 10, according to each of Examples 1 to 6 more specifically showing Embodiment, will be described with reference to the drawings. In Examples 1 to 6, the configurations omitted in Embodiment will be focused on. The same configurations in Embodiment will be designated by the same reference numerals and the description thereof will be omitted.

Example 1

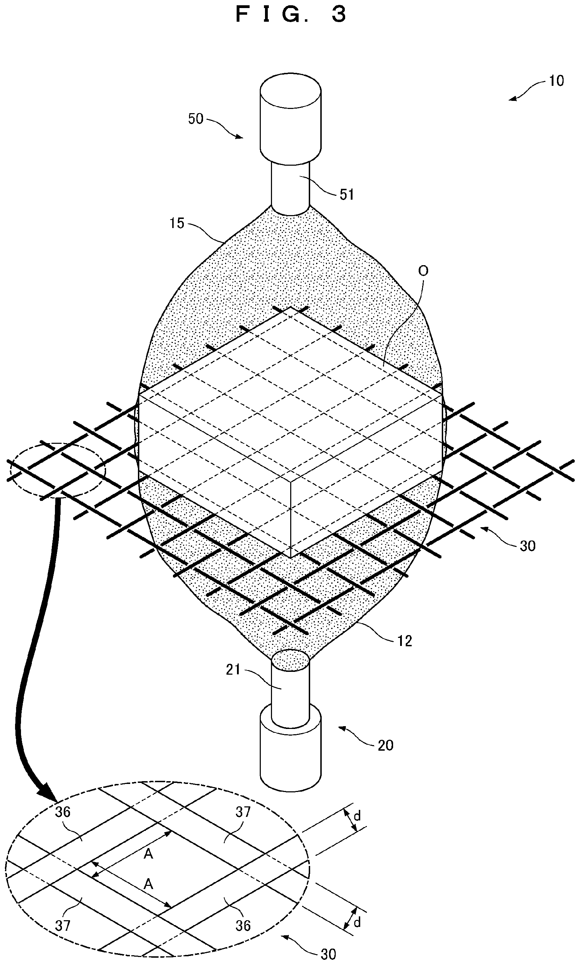

As illustrated in FIG. 3, the electron beam sterilizer 10 of Example 1 includes a mesh 30, a lower electron beam irradiator 20, and an upper electron beam irradiator 50. The sterilization object O is placed on the fixed mesh 30. The lower electron beam irradiator 20 irradiates the sterilization object O on the mesh 30 with the electron beam 12 from a lower side through the mesh 30. The upper electron beam irradiator 50 irradiates the sterilization object O with an electron beam 15 from un upper side.

The mesh 30 includes longitudinal wire rods 36 and lateral wire rods 37 woven together. Each of the longitudinal wire rods 36 and the lateral wire rods 37 has a circular cross section. The curved surfaces 4 thereof come into contact with the sterilization object O to be placed on the mesh 30. Additionally, the longitudinal wire rods 36 and the lateral wire rods 37 cross each other. That is, one of the wire rods 36 and 37 is under the other of wire rods 36 and 37 at each of the crossing parts in these wire rods 3. The sterilization object O is not in contact with the under parts and areas close to the under parts. In other words, the mesh 30 is made by weaving the longitudinal wire rods 36 and the lateral wire rods 37. Therefore, the contact parts with the sterilization object O to be placed become smaller.

To enhance the effect of sterilization, a diameter d of each of the longitudinal wire rods 36 and the lateral wire rods 37 is preferably small. However, the smaller diameter d causes the mesh 30 difficult to immovably hold the sterilization object O. This is because of the relationship between a strength of the wire rods 3 and a weight of the sterilization object O. Therefore, the diameter d is large enough to immovably hold the sterilization object O. Similarly, an opening ratio of the mesh 30 is preferably large to enhance the effect of sterilization. However, the larger opening ratio causes the mesh 30 difficult to immovably hold the sterilization object O. This is because of the relationship between the strength of the wire rods 3 and the weight of the sterilization object O. Therefore, the opening ratio is set to a ratio enough to immovably hold the sterilization object O. Note that the opening ratio $\varepsilon$ is calculated from the following equation (1).

$$\varepsilon = \{A/(A+d)\}^2 \times 100 \quad \cdots \cdots \cdots \cdots (1)$$

where:
the opening ratio is $\varepsilon$, the opening is A, and the diameter of each of the wire rods 36 and 37 is d.

Here, acceleration voltages of the lower electron beam irradiator 20 and the upper electron beam irradiator 50 are set to 80 kV to 150 kV. Energy of the electrons 1 (primary electrons 1) of the electron beam 12 is set to 80 keV to 150 keV. Assuming that such electrons 1 are emitted to a plate obtained by arranging the wire rods having a wire diameter of 0.03 mm side by side without gaps, these electrons 1 have low energy so as not to pass through the plate. The sterilization object O is sterilized by such electrons 1 coming around the wire rods 3 from the openings of the mesh 30, as well as the secondary electrons 2.

The lower electron beam irradiator 20 and the upper electron beam irradiator 50 are nozzle type electron beam irradiators having nozzles 21 and 51, respectively. The nozzle type electron beam irradiators emit the electron beams 12 and 15 from tips of the nozzles 21 and 51, respectively. This allows the nozzle type electron beam irradiator to emit an electron beam having a cross section smaller than a non-nozzle type electron beam irradiator. Therefore, the nozzle type electron beam irradiators are suitably used as the lower electron beam irradiator 20 and the upper electron beam irradiator 50 for the small sterilization object O, as illustrated in FIG. 3. However, for the larger sterilization object O, the non-nozzle type electron beam irradiators (not shown) are suitably used as the lower electron beam irradiator 20 and the upper electron beam irradiator 50. This is because the non-nozzle type electron beam irradiator emits an electron beam having a large cross section. In such electron beam sterilizer 10, the upper electron beam irradiator 50 enables sterilization of a part that cannot be sterilized by the lower electron beam irradiator 20 alone (the upper half of the sterilization object O) by the irradiation with the electron beam 15.

The electron beam sterilizer 10 of Example 1 can obtain the same effect as the electron beam sterilizer 10 of Embodiment. Moreover, a sufficient number of the primary electrons 1 and secondary electrons 2 are hardly blocked by the mesh 30. Thus, sterilization effect can be further enhanced.

Here, the mesh 30 is not particularly limited as long as a material thereof is not deteriorated by the irradiation with the electron beam 12. The mesh 30 is preferably made of a metal such as stainless steel or titanium. Further, the mesh 30 is preferably grounded in order to prevent charging.

Example 2

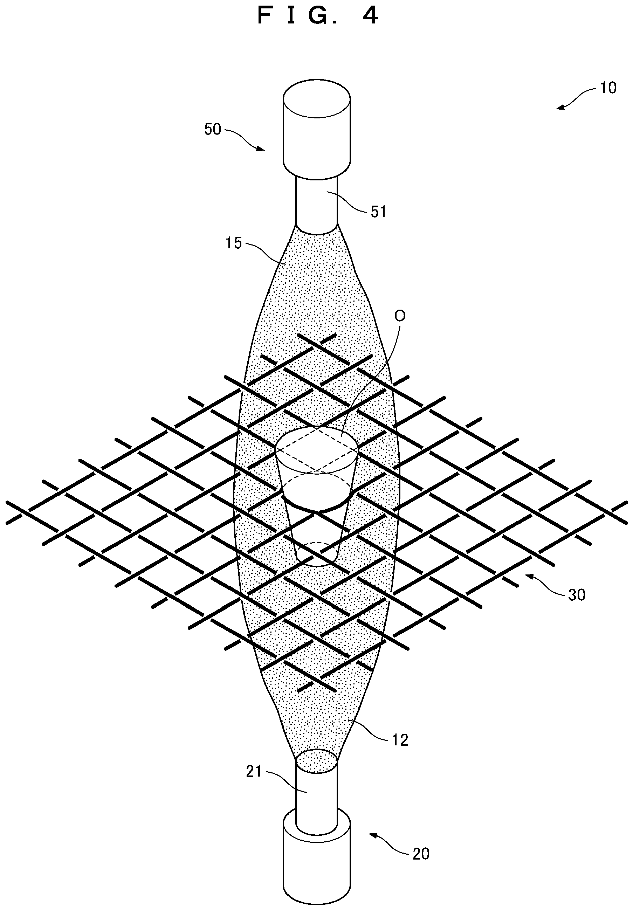

The electron beam sterilizer 10 of Example 2 has the same configuration as the electron beam sterilizer 10 of Example 1, as illustrated in FIG. 4. However, the electron beam sterilizer 10 of Example 2 differs from the electron beam sterilizer 10 of Example 1 in holding the sterilization object O not by placing the sterilization object O on the mesh 30 but by inserting the sterilization object O in the mesh 30.

The mesh 30 of the electron beam sterilizer 10 in Example 2 has the openings of the mesh 30 of the electron beam sterilizer 10 in Example 1. Additionally, the openings of the mesh 30 are large enough to insert the sterilization object O therein.

The mesh 30 holds the sterilization object O by inserting the sterilization object O in one of the openings. Therefore, only a part of the wire rods 36 and 37 of such opening then comes into contact with the sterilization object O. Therefore, the contact part of the mesh 30 and the sterilization object O is extremely small.

The electron beam sterilizer 10 of Example 2 can obtain the same effect as the electron beam sterilizer 10 of Embodiment. Moreover, a sufficient number of the primary electrons 1 and secondary electrons 2 are hardly blocked by the mesh 30. Thus, sterilization effect can be further enhanced.

Example 3

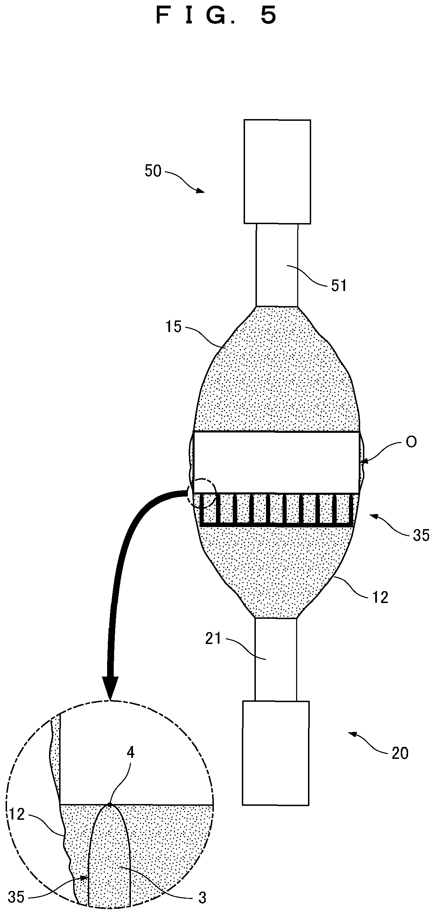

The electron beam sterilizer 10 of Example 3 includes a brush member 35, which is also called a pin-up, instead of the mesh 30 of Examples 1 and 2, as illustrated in FIG. 5.

The brush member 35 includes a large number of wire rods 3 arranged in a brush shape. The brush member 35 holds the sterilization object O by placing the sterilization object O on tips of the wire rods 3. Each of the tips of the wire rods 3 has a rounded shape rather than a sharp shape, that is, the curved surface 4. The sterilization object O is placed on such tips of the wire rods 3. Therefore, the contact parts of the wire rods 3 and the sterilization object O are extremely small. Additionally, the wire rods 3 extend along a direction in which the electron beam 12 is emitted from the lower electron beam irradiator 20. Consequently, the electrons 1 of the electron beam 12 are hardly blocked.

The electron beam sterilizer 10 of Example 3 can obtain the same effect as the electron beam sterilizer 10 of Embodiment. Moreover, a sufficient number of the primary electrons 1 and secondary electrons 2 are hardly blocked by the wire rods 3. Thus, sterilization effect can be further enhanced.

Example 4

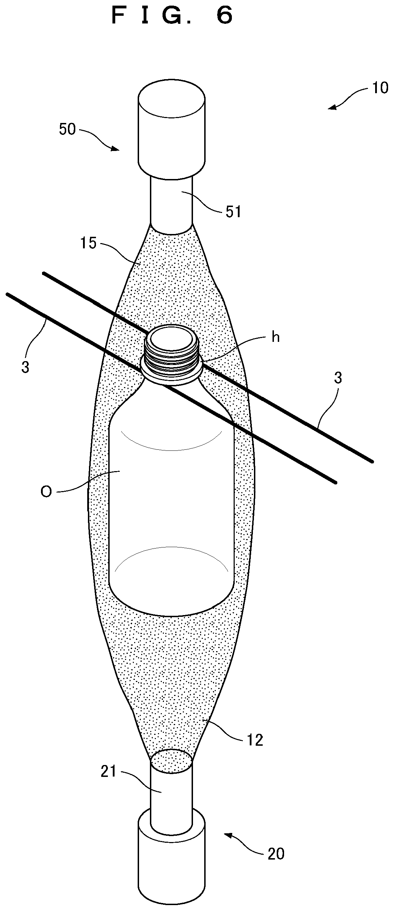

The electron beam sterilizer 10 of Example 4 holds the sterilization object O by hanging, as illustrated in FIG. 6, unlike the electron beam sterilizers 10 described in Examples 1 to 3, which hold the sterilization object O by placing or inserting.

Each of the wire rods 3 used for hanging has a circular cross section. The sterilization object O is hung to be held between the wire rods 3. Therefore, the contact parts of the wire rods 3 and the sterilization object O are extremely small. Further, the sterilization object O may include a portion h hung on the wire rods 3 like a plastic bottle O in FIG. 6. The sterilization object O preferably includes the portion h to be hung above the center of gravity. This allows the wire rods 3 to stably hold the sterilization object O by hanging.

The electron beam sterilizer 10 of Example 4 can obtain the same effect as the electron beam sterilizer 10 of Embodiment. Moreover, a sufficient number of the primary electrons 1 and secondary electrons 2 are hardly blocked by the wire rods 3. Thus, sterilization effect can be further enhanced.

Example 5

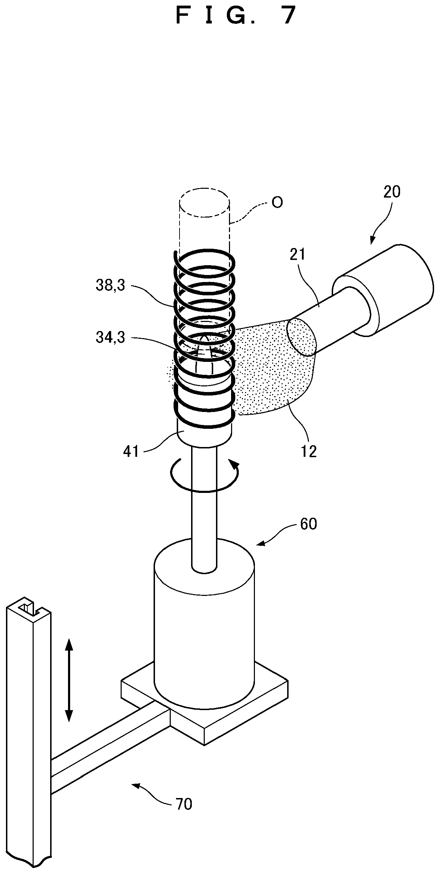

The electron beam sterilizer 10 of Example 5 includes a needle member 34 and a circular member 38, as illustrated in FIG. 7. The needle member 34 holds the sterilization object O by placing the sterilization object O on a tip thereof. The circular member 38 holds the sterilization object O by surrounding a side surface of the sterilization object O on the tip of the needle member 34. The needle member 34 and the circular member 38 include the wire rods 3.

The needle member 34 is attached to an upper surface of a cylindrical member 41. The cylindrical member 41 defines an inner diameter of the circular member 38. The circular member 38 is formed in a coil shape extending upward from the cylindrical member 41. However, the circular member 38 is not limited to the coil shape. The circular member 38 can be any shape capable of surrounding the side surface of the sterilization object O. The circular member 38 may have the inner diameter equal to or less than an outer diameter of the sterilization object O. The sterilization object O to be surrounded is inserted into such circular member 38. Contrarily, the inner diameter may exceed the outer diameter of the sterilization object O. The circular member 38 with such inner diameter allows the sterilization object O inside thereof to lean against the circular member 38.

The electron beam sterilizer 10 of Example 5 includes a rotating mechanism 60 and an elevating mechanism 70. The rotating mechanism 60 rotates the sterilization object O with respect to the electron beam irradiator 20 together with the wire rod 3 (the needle member 34 and the circular member 38). The elevating mechanism 70 moves the sterilization object O and the wire rod 3 up and down with respect to the electron beam irradiator 20.

In such electron beam irradiator 20, the tip of the nozzle 21 emitting the electron beam 12 faces the side surface of the sterilization object O at the lower end of the sterilization object O. The electron beam irradiator 20 irradiates the side surface of the sterilization object O over the circular member 38 with the electron beam 12 emitted from the electron beam irradiator 20. The electron beam irradiator 20 also irradiates a bottom surface of the sterilization object O, over the needle member 34, with the electron beam 12 coming around to the lower part of the sterilization object O.

Next, a method of using the electron beam sterilizer 10 of Example 5 will be described.

First, the circular member 38 and the needle member 34 hold the sterilization object O by inserting the sterilization object O into the circular member 38 and placing the sterilization object O on the tip of the needle member 34.

Then, the sterilization object O is irradiated with the electron beam 12 emitted from the electron beam irradiator 20, while the sterilization object O rotates and moves up and down together with the needle member 34 and the circular member 38. The ascent and descent of the sterilization object O enables sterilization, by the irradiation of the electron beam 12, of the side and bottom surfaces of the sterilization object O, as well as the other side surface (i.e., the entire surface). Additionally, the rotation of the sterilization object O enables uniform sterilization.

Regarding the rotation and the ascent/descent, the sterilization object O may move up and down, while rotating. The rotation and the ascent/descent may be repeated alternately. Alternately repeating the rotation and the ascent/descent can simplify control by the rotating mechanism 60 and the elevating mechanism 70. Preferably, the ascent/descent by the elevating mechanism 70 is stopped at 2 to 6 of predetermined points. Then, the rotating mechanism 60 rotates the sterilization object O 3 to 5 times (that is, 3 to 5 rotations) at each stop. More preferably, to sufficiently sterilize the bottom surface of the sterilization object O, irradiation time is increased at a height of the points where the electron beam 12 comes around to the bottom surface of the sterilization object O compared with the other heights of the points.

The electron beam sterilizer 10 of Example 5 can obtain the same effect as the electron beam sterilizer 10 of Embodiment. Moreover, the entire surface of the sterilization object O can be sterilized.

Example 6

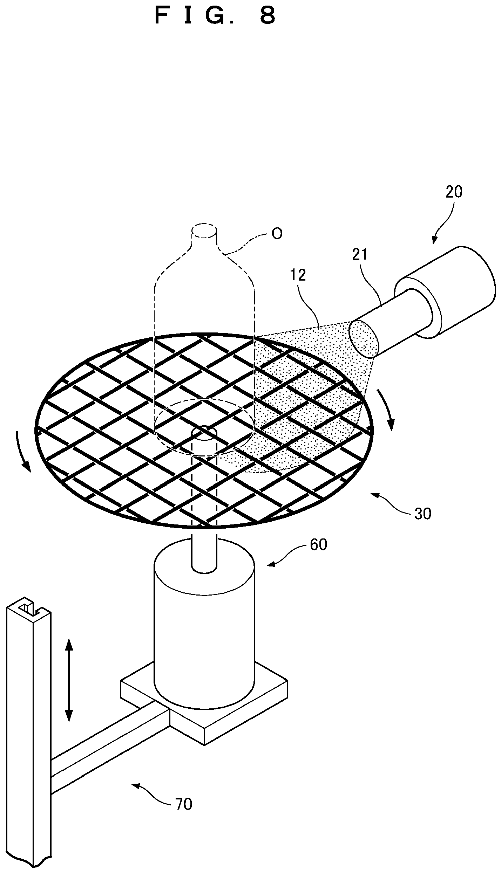

The electron beam sterilizer 10 of Example 6 includes the mesh 30 as the wire rod 3, as illustrated in FIG. 8. That is, the mesh 30 is substituted for the needle member 34 and the circular member 38 of Example 5.

The mesh 30 does not surround the side surface of the sterilization object O. This allows the electron beam sterilizer 10 to sterilize the sterilization object O having a large outer diameter such as a plastic bottle.

The electron beam sterilizer 10 of Example 6 can obtain the same effect as the electron beam sterilizer 10 of Example 5. Moreover, the sterilization object O having a large outer diameter can also be sterilized.

Here, Embodiment shows two cross sections of the wire rods 3 in FIG. 1, but not necessarily limited to the plurality of the wire rods 3. The single wire rod 3 may be used as long as the wire rod 3 can hold the sterilization object O with one wire.

Further, Embodiment shows the circular cross sections of the wire rods 3 in FIG. 1. However, the shape is not limited to a circle as long as the part in contact with the sterilization object O is curved.

Furthermore, Embodiment describes that the secondary electrons 2 are emitted from the sterilization object O. However, the secondary electrons 2 include electrons emitted from the wire rods 3.

Furthermore, Embodiment and Examples 1 to 6 describe that the relative position of the wire rod 3 with respect to the sterilization object O is constant. This also includes the meaning of natural movement of the wire rod 3 and the sterilization object O by deformation due to increased temperature caused by irradiation of the electron beams 12 and 15.

Furthermore, Examples 1 to 6 describe the nozzle type electron beam irradiator or the non-nozzle type electron beam irradiator as the electron beam irradiator 20. The electron beam irradiator 20 may be a scan type electron beam irradiator. The scan type electron beam irradiator emits an electron beam having a narrow width. Such scan type electron beam irradiator irradiates the sterilization object O, while scanning with the electron beam in a vertical direction. This can obtain the same effect as the electron beam sterilizer having the elevating mechanism 70.

Furthermore, Embodiment and Examples 1 to 4 do not describe the details of what the wire rods 3, the mesh 30, and the brush member 35 are fixed to. The wire rods 3, the mesh 30, and the brush member 35 are fixed to at least something such as an inner wall of a box body in which the electron beams 12 and 15 are irradiated.

Furthermore, Examples 1 to 4 describe that the wire rods 3 immovably hold the sterilization object O. Therefore, a drive system for moving the wire rods 3 is not required, enhancing the sterilization effect.

Furthermore, Examples 1 and 2 describe that the mesh 30 includes the longitudinal wire rods 36 and the lateral wire rods 37 woven together. However, instead of such mesh 30, only the longitudinal wire rods 36 or only the lateral wire rods 37 may be arranged in parallel for the mesh 30.

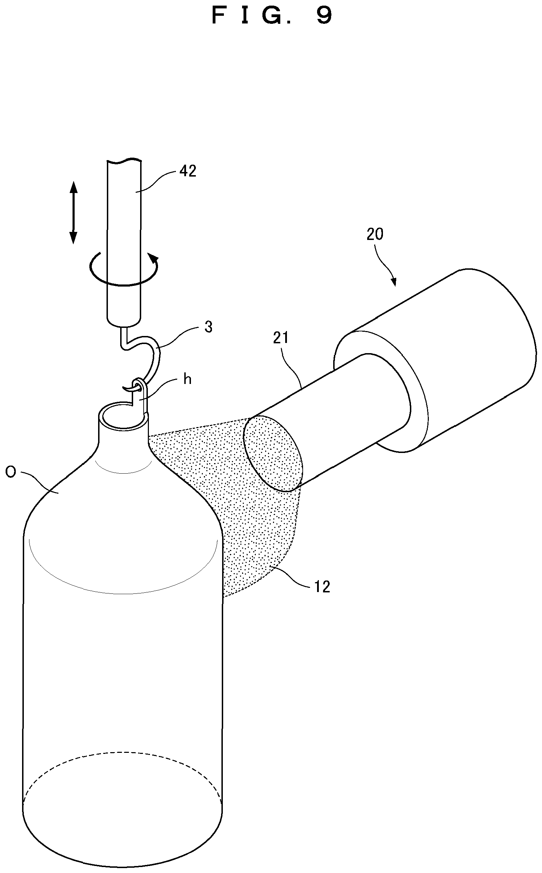

Furthermore, Example 4 shows that the sterilization object O is hung on the two wire rods 3 in FIG. 6. However, as illustrated in FIG. 9, the sterilization object O may include a tab h having a hole. The single wire rod 3 of a hook shape extending from a hanging tool 42 may be hooked in the hole of the tab h.

Furthermore, Embodiment and Examples 1 to 4 does not describe the rotating mechanism 60 described in Examples 5 and 6. However, the electron beam sterilizers 10 in Examples 1 to 4 can obviously include the rotating mechanism 60.

Furthermore, Embodiment and Examples 1 to 6 are merely exemplary and are not restrictive in all the aspects. The scope of the present invention is not indicated by the foregoing description but the claims. The scope of the present invention is intended to include meanings equivalent to the claims and all changes in the scope. From among the configurations described in Embodiment and Examples 1 to 6, the configurations other than those described as a first invention in "Solution to Problem" are optional and thus can be deleted and changed as appropriate.

**Claims**

1. An electron beam sterilizer comprising:

   at least one wire rod configured to hold a sterilization object, the wire rod being in a constant relative position with respect to the sterilization object; and
   an electron beam irradiator configured to irradiate the sterilization object held by the wire rod with an electron beam over the wire rod,
   wherein the wire rod includes a curved surface in contact with the sterilization object to hold, and
   electrons of the electron beam from the electron beam irradiator, and secondary electrons excited by the electrons come around to the curved surface in contact with the sterilization object to sterilize the sterilization object.

2. The electron beam sterilizer according to claim 1, further comprising a mesh configured to hold the sterilization object by placing the sterilization object on the mesh, or inserting the sterilization object in the mesh, wherein the mesh includes the wire rod woven together.

3. The electron beam sterilizer according to claim 1, further comprising a brush member configured to hold the sterilization object by placing the sterilization object on the brush member, wherein the brush member includes the wire rod extending along a direction in which the electron beam is emitted from the electron beam irradiator.

4. The electron beam sterilizer according to claim 1, wherein the wire rod holds the sterilization object by hanging the sterilization object.

5. The electron beam sterilizer according to claim 1, further comprising:

   a needle member configured to hold the sterilization object by placing the sterilization object on a tip of the needle member; and
   a circular member configured to hold the sterilization object by surrounding a side surface of the sterilization object on the tip of the needle member,
   wherein the needle member and the circular member include the wire rod.

6. The electron beam sterilizer according to any one of claims 1 to 5, further comprising a rotating mechanism configured to rotate the sterilization object and the wire rod with respect to the electron beam irradiator.

7. The electron beam sterilizer according to claim 6, wherein the electron beam irradiator irradiates the side surface of the sterilization object with the electron beam.

8. The electron beam sterilizer according to claim 7, further comprising an elevating mechanism configured to move the sterilization object and the wire rod up and down with respect to the electron beam irradiator.

# F I G. 1

# F I G.　2

# F I G. 3

# F I G. 4

# F I G． 5

# FIG. 6

# F I G. 7

# FIG. 8

# F I G. 9

## INTERNATIONAL SEARCH REPORT

| International application No. |
| --- |
| PCT/JP2020/021127 |

**A.  CLASSIFICATION OF SUBJECT MATTER**
A61L 2/08(2006.01)i
FI: A61L2/08 108

According to International Patent Classification (IPC) or to both national classification and IPC

**B.  FIELDS SEARCHED**

Minimum documentation searched (classification system followed by classification symbols)
A61L2/08; G21K5/10

Documentation searched other than minimum documentation to the extent that such documents are included in the fields searched
Published examined utility model applications of Japan      1922–1996
Published unexamined utility model applications of Japan    1971–2020
Registered utility model specifications of Japan            1996–2020
Published registered utility model applications of Japan    1994–2020

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | JP 2003-161800 A (MITSUBISHI ELECTRIC CORP.)<br>06.06.2003 (2003-06-06) claims 1-2, paragraphs<br>[0001], [0015]-[0016], [0022]-[0023], [0025]-<br>[0029], fig. 1, 3 | 1-2, 4, 6-7<br>8<br>3, 5 |
| X | JP 2003-014900 A (IWASAKI ELECTRIC CO., LTD.)<br>15.01.2003 (2003-01-15) claim 1, paragraphs<br>[0001], [0011]-[0013], [0016], fig. 4 | 1-2 |
| Y | WO 2017/159200 A1 (HITACHI ZOSEN CORP.) 21.09.2017<br>(2017-09-21) paragraphs [0038]-[0042], fig. 8 | 8 |
| A | JP 11-019190 A (IWASAKI ELECTRIC CO., LTD.)<br>26.01.1999 (1999-01-26) entire text | 1-8 |

☒  Further documents are listed in the continuation of Box C.      ☒  See patent family annex.

| | |
| --- | --- |
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| 07 August 2020 (07.08.2020) | 18 August 2020 (18.08.2020) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

International application No.

PCT/JP2020/021127

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2018-070199 A (HITACHI ZOSEN CORP.) 10.05.2018 (2018-05-10) entire text | 1-8 |

Form PCT/ISA/210 (continuation of second sheet) (January 2015)

**INTERNATIONAL SEARCH REPORT**
Information on patent family members

International application No.

PCT/JP2020/021127

| Patent Documents referred in the Report | Publication Date | Patent Family | Publication Date |
|---|---|---|---|
| JP 2003-161800 A | 06 Jun. 2003 | (Family: none) | |
| JP 2003-014900 A | 15 Jan. 2003 | (Family: none) | |
| WO 2017/159200 A1 | 21 Sep. 2017 | JP 2017-165474 A EP 3431402 A1 paragraphs [0042]-[0047], fig. 8 | |
| JP 11-019190 A | 26 Jan. 1999 | (Family: none) | |
| JP 2018-070199 A | 10 May 2018 | WO 2018/105578 A1 | |

Form PCT/ISA/210 (patent family annex) (January 2015)

**REFERENCES CITED IN THE DESCRIPTION**

**Patent documents cited in the description**

- JP 11019190 A